# EUROPEAN PATENT APPLICATION

(11) **EP 2 075 035 A1**
(43) Date of publication of application: **01.07.2009**
(21) Application number: 08253996.6
(22) Date of filing: 12.12.2008
(51) Int. Cl.: A61N 5/06, A61M 21/00

(54) **Photic stimulation for eyes**

(30) Priority: 24.12.2007 GB 0725124
(71) Applicant: Carr, Peter, Ardross Ross-shire IV7 0XN (GB)
(72) Inventor: Carr, Peter, Ardross Ross-shire IV7 0XN (GB)
(74) Representative: Peter, Kenneth William

(57) **Abstract**

The present invention relates to photic stimulation apparatus 10 for stimulating the eyes of a subject. The apparatus comprises at least one light source 26, 28 that is operable to emit light, which, in use of the apparatus, is seen by each of the right and left eyes of the subject alternately and such that the light is seen at least mainly by one of the eyes each alternation. The apparatus also comprises a controller operable: to provide light from the at least one light source to the right and left eyes alternately; and to pulse the at least one light source 26, 28 repeatedly at a frequency of between substantially 36 Hz and substantially 44 Hz when the at least one light source is seen by each eye and during each alternation. The photic stimulation apparatus is configured such that in use the photic stimulation apparatus is spaced apart from the eyeballs of the subject.

## Description

### Field of the invention

The present invention relates to photic stimulation apparatus that is operable to stimulate movement of the eyes of a subject using the apparatus.

### Background to the invention

Eye Movement Desensitisation and Reprocessing (EMDR) is a known technique that has been used to resolve stress related disorders, such as Post Traumatic Stress Disorder. Photic stimulation apparatus has been used in EMDR therapy. For example, Neurolateral Inc. of Kingston, Canada provides Personal Computer (PC) operable software, which causes a PC to display a moving image that the user follows with his or her eyes to thereby provide the necessary eye movement. A further example is the Eyescan® apparatus by Neurotek® Corporation of Wheat Ridge, Colorado, which comprises a plurality of light sources spaced apart along an elongate member. In use, the elongate member is disposed such that it extends from left to right in front of a user and the light sources are actuated in turn from left to right and vice-versa. A user follows the light sources with his or her eyes as they are actuated to thereby effect the necessary eye movement.

The present inventor has appreciated that known photic stimulation apparatus, such as the EMDR apparatus described above, have shortcomings.

It is therefore an object for the present invention to provide improved apparatus for photic stimulation of a user's eyes.

### Statement of invention

The present invention has been devised in view of shortcomings of known apparatus. Thus according to a first aspect of the present invention, there is provided photic stimulation apparatus for stimulating the eyes of a subject, the apparatus comprising:
at least one light source that is operable to emit light, which, in use of the apparatus, is seen by each of the right and left eyes of the subject alternately and such that the light is seen at least mainly by one of the eyes each alternation; and
a controller operable: to provide light from the at least one light source to the right and left eyes alternately; and to pulse the at least one light source repeatedly at a frequency of between substantially 36 Hz and substantially 44 Hz when the at least one light source is seen by each eye and during each alternation,
the photic stimulation apparatus being configured such that in use the photic stimulation apparatus is spaced apart from the eyeballs of the subject.

In use, operation of the controller such that light is seen mainly by each of the left and right eyes alternately can stimulate the user to move his or her eyeballs. Configuring the apparatus such that the photic stimulation apparatus is spaced apart from the eyeballs of the subject during use allows for free, unhindered movement of the eyeballs. The light is seen at least mainly by each eye. This means that when the light is seen mainly by the right eye the light is seen to a lesser extent by the left eye. Nevertheless, one eye seeing the light mainly vis-à-vis the other eye is normally sufficient to stimulate eye movement. Repeated pulsing of the light source when the light source is seen by each eye (i.e. repeated pulsing for the left eye, then repeated pulsing for the right eye, etc.) provides a second means of stimulation.

The inventor has found that the combined use of the two means of stimulation (i.e. repeated pulsing for each eye and alternation between left and right eyes) provides for improved effectiveness of therapy. It is understood that the alternation between left and right eyes can provide for equal arousal levels in the left and right brain hemispheres and for inter-hemispherical integration. It is understood that the repeated pulsing can regulate cognitive processing. Together, i.e. the alternation and pulsing being simultaneous, the two means of stimulation can provide for an improvement in emotional processing.

More specifically, repeated pulsing of the at least one light source may be at a frequency of substantially 40 Hz. A frequency of substantially 40 Hz has been found to promote psychological integration within the brain and the nervous system.

Alternatively or in addition, the controller may be operable to provide light from the at least one light source to the right and left eyes alternately at a frequency of between substantially 0.25 Hz and substantially 5 Hz.

More specifically, the controller may be operable to provide light from the at least one light source to the right and left eyes alternately at a frequency of less than substantially 2 Hz. A frequency of less than substantially 2 Hz has been found to be reasonably comfortable for a user when involved in eye movement.

More specifically, the controller may be operable to provide light from the at least one light source to the right and left eyes alternately at a frequency of substantially 1 Hz. A frequency of substantially 1 Hz has been found to be comfortable for a user when involved in eye movement and appropriate for association with less than positive thoughts.

More specifically, the controller may be operable to provide light from the at least one light source to the right and left eyes alternately at a frequency of substantially 0.5 Hz. A frequency of substantially 0.5 Hz has been found to be comfortable for a user when involved in eye movement and appropriate for association with positive thoughts.

Alternatively or in addition, the controller may be user operable to change at least one of an alternation frequency and a repeated pulse frequency, e.g. by selecting at least one of an alternation frequency and a repeated pulse frequency from a plurality of frequencies.

Alternatively or in addition, the apparatus may comprise: at least one first light source that is operable to emit light, which, in use of the apparatus, is seen at least mainly by a right eye of the right and left eyes of the subject; and at least one second light source that is operable to emit light, which, in use of the apparatus, is seen at least mainly by a left eye of the right and left eyes of the subject.

More specifically, the controller may be operable: to actuate the at least one first light source and the at least one second light source alternately; and to pulse an at least one light source repeatedly when the at least one light source is actuated,

Alternatively or in addition, the photic stimulation apparatus may be configured such that, in use, the at least one first light source is disposed in front of the right eye of the user and the at least one second light source is disposed in front of the left eye of the user.

More specifically, the photic stimulation apparatus may be configured such that, in use, each of the at least one first and second light sources is disposed in front of the right eye of the user distally of the mid-sagittal plane of the user.

More specifically, the photic stimulation apparatus may be configured such that, in use, each of the at least one first and second light sources is disposed at a lateral periphery of the user's visual field.

More specifically, the photic stimulation apparatus may be configured such that, in use, light sources are disposed in the user's visual field solely at the lateral periphery of the user's visual field. Having light sources disposed in the user's visual field solely at the lateral periphery of the user's visual field may provide for ease of cuing of side to side movement of the user's eyes.

Alternatively or in addition, the at least one light source may comprise a Light Emitting Diode (LED), such as a high brightness LED.

Alternatively or in addition, the photic stimulation apparatus may comprise a support arrangement on which the at least one light source is mounted.

More specifically, the support arrangement may be rigid.

Alternatively or in addition, the support arrangement may be at least one of transparent and translucent. In use, a transparent or translucent support arrangement enables a party other than the user, e.g. a therapist, to have a relatively clear view of the user's facial expression. Thus, the therapist can user the user's facial expressions to, for example, direct the course of an EMDR therapy session.

Alternatively or in addition, the support arrangement may be configured to be mounted on the head of a user.

More specifically, the support arrangement may comprise glasses.

More specifically, the photic stimulation apparatus may comprise: at least a first light source mounted on a first leg of the glasses, the first leg being configured to engage with a right ear of the user; and at least a second light source mounted on a second leg of the glasses, the second leg being configured to engage with a left ear of the user.

More specifically, each of the at least one first and second light sources may be mounted on its respective leg such that, in use, light is emitted from the first and second light sources away from the eyes of a user.

Alternatively or in addition, the glasses may be formed at least in part of a plastics material.

Alternatively or in addition, a reflective component may be mounted over each of a right lens and a left lens of the glasses such that a part of a field of view through each of the right and left lenses is obscured. The light sources may be disposed on support apparatus such that light is emitted from the light sources towards the reflective components.

More specifically, the reflective component may be a film of reflective material attached to a lens. The reflective component may have dimensions of substantially 15 mm by substantially 40 mm and may be oriented such that, in use, the 15 mm dimension extends generally laterally of a user's eyes.

Alternatively or in addition, the reflective component may be mounted such that it extends over a portion of a lens distally of a mid-sagittal plane of the user.

Alternatively or in addition, the reflective component may be mounted such that it extends over a portion of a lens at a lateral periphery of the user's visual field.

Alternatively or in addition, the controller may comprise a safety switch having a first state, in which no light is emitted from the at least one light source, and a second state, in which light is emitted from the at least one light source.

More specifically, the safety switch may be configured for actuation by contact with a person, e.g. by a user's touch.

More specifically, the safety switch may be configured such that contact with the person needs to be maintained to maintain the switch in the second state. Thus, in the absence of contact the switch may change from the second state to the first state. In use, the safety switch provides for safe operation. For example, the need to have contact with the switch provides of ease of discontinuation of therapy, e.g. if the user feels discomfort, and reduces the likelihood of improper use of the apparatus, e.g. by children.

Alternatively or in addition, the safety switch may comprise a touch sensor that is operative when touched by a person, such as the user.

More specifically, the touch sensor may comprise a capacitive sensor.

According to a second aspect of the present invention, there is provided photic stimulation apparatus for stimulating the eyes of a subject, the apparatus comprising:
at least one light source that is operable to emit pulsed light; and a controller operable to control emission of light from the at least one light source such that pulses of emitted light are seen by at least one of the right and left eyes of the subject; and
a safety switch having a first state, in which no light is emitted from the at least one light source, and a second state, in which light is emitted from the at least one light source, the safety switch being configured for actuation by contact with a person and such that contact with the person is required to maintain the switch in the second state.

More specifically, the apparatus may be configured and the controller may be operable to control emission of light from the at least one light source such that the emitted light is seen by each of the right and left eyes of the subject alternately and such that the light is seen at least mainly by one of the eyes each alternation.

More specifically, the controller may be operable to pulse the at least one light source repeatedly when the at least one light source is seen by each eye.

Alternatively or in addition, the photic stimulation apparatus may be configured such that in use the photic stimulation apparatus is spaced apart from the eyeballs of the subject.

Further embodiments of the second aspect of the present invention may comprise one or more features of the first aspect of the present invention.

According to a further aspect of the present invention there is provided photic stimulation apparatus for stimulating the eyes of a subject, the apparatus comprising: at least one light source that is operable to emit light; and a controller operable to control emission of light from the at least one light source such that the pulses of emitted light are seen by at least one of the right and left eyes of the subject.

More specifically, the apparatus may be configured and the controller may be operable to control emission of light from the at least one light source such that the emitted light is seen by each of the right and left eyes of the subject alternately and such that the light is seen at least mainly by one of the eyes each alternation.

More specifically, the controller may be operable to pulse the at least one light source repeatedly when the at least one light source is seen by each eye. It is understood that the repeated pulsing can regulate cognitive processing, e.g. a slower pulse frequency can induce a relaxed state of mind and a faster pulse frequency can induce an alert state of mind.

More specifically, repeated pulsing of the at least one light source may be at a frequency of less than substantially 50 Hz.

Alternatively or in addition, repeated pulsing of the at least one light source may be at a frequency of more than substantially 5 Hz.

Alternatively or in addition, repeated pulsing of the at least one light source may be at a frequency of between substantially 10 Hz and substantially 20 Hz. The frequency range of substantially 10 Hz to substantially 20 Hz has been found to promote relaxation.

Alternatively or in addition, repeated pulsing of the at least one light source may be at a frequency of between substantially 20 Hz and substantially 40 Hz. The frequency range of substantially 20 Hz to substantially 40 Hz has been found to promote cognitive activity.

Alternatively or in addition, the photic stimulation apparatus may be configured such that in use the photic stimulation apparatus is spaced apart from the eyeballs of the subject.

Further embodiments of the further aspect of the present invention may comprise one or more features of any previous aspect of the present invention.

### Brief description of the drawings

Further features and advantages of the present invention will become apparent from the following specific description, which is given by way of example only and with reference to the accompanying drawings, in which:
Figure 1 a provides a front view of glasses according to the present invention;
Figure 1b provides a plan view of the glasses of Figure 1a;
Figure 2 provides a view of a controller according to the present invention; and
Figure 3 is a circuit schematic of operative electronic components of the controller and the glasses shown in Figures 1a, 1b and 2.

### Specific description

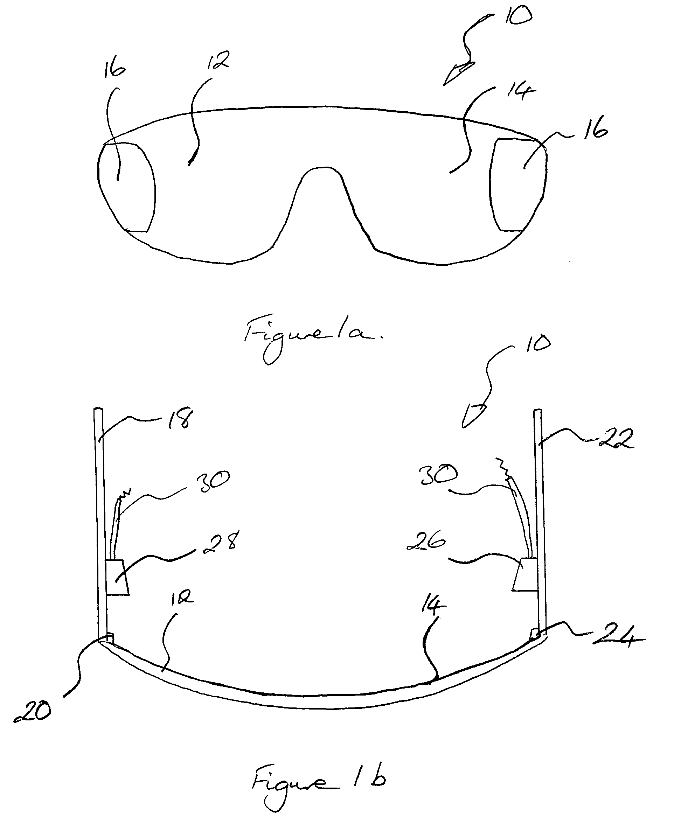

Figure 1 a provides a front view of glasses 10 according to the invention. The glasses 10 (which constitute a support arrangement) comprise left 12 and right 14 lenses. The glasses are formed of a rigid plastics material by a well known process. A film of reflective material 16 (which constitutes a reflective component) is attached to each of the left and right lenses.
Each film of reflective material is located distally of a mid-sagittal plane (which is defined by a line bisecting the glasses into left and right lens parts) such that the film of reflective material covers a peripheral portion of the lens. Each film of reflective material has dimensions of substantially 40 mm by substantially 15 mm.

Figure 1b provides a plan view of the glasses 10 shown in Figure 1a. As can be seen from Figure 1b, the glasses comprise a left leg 18 that is attached by a hinge 20 at the left lens 12 of the glasses and a right leg 22 that is attached by a hinge 24 at the right lens 14 of the glasses. The left and right legs 18, 22 are of conventional design such that, in use, each engages with an ear of a user of the glasses. A first high brightness white Light Emitting Diode (LED) 26 is attached to the right leg 22 and a second white high brightness Light Emitting Diode (LED) 28 is attached to the left leg 18. The first and second LEDs 26, 28 are attached to their respective legs such that light emitted by the LEDs is directed towards the film of reflective material provided on the respective lens 14, 12. In use, the films of reflective material reflect light emitted by the LEDs towards a user's eyes. Electrical power is conveyed to each LED 26, 28 by way of a signal cable 30. The LEDs are 100mA 10mm series high intensity LED lamps (part numbers 850-248, 250, 252, 254, 255) from JPR Electronics of Dunstable, Bedfordshire, United Kingdom.

In one form the glasses 10 of Figures 1 a and 1 b are formed of clear plastics. This form of the glasses is suitable for use in supervised therapy sessions because the therapist has a generally clear view of the user's facial expressions and can therefore take account of facial expressions when directing the course of the therapy. In another form, frames of the glasses are formed of opaque plastics material and may be of a shape and configuration that cover a greater part of the face of the user than the other form. Such other form of the glasses is suitable for use by a user of the apparatus in unsupervised therapeutic sessions where clear sight of the user's facial expressions is of no importance.

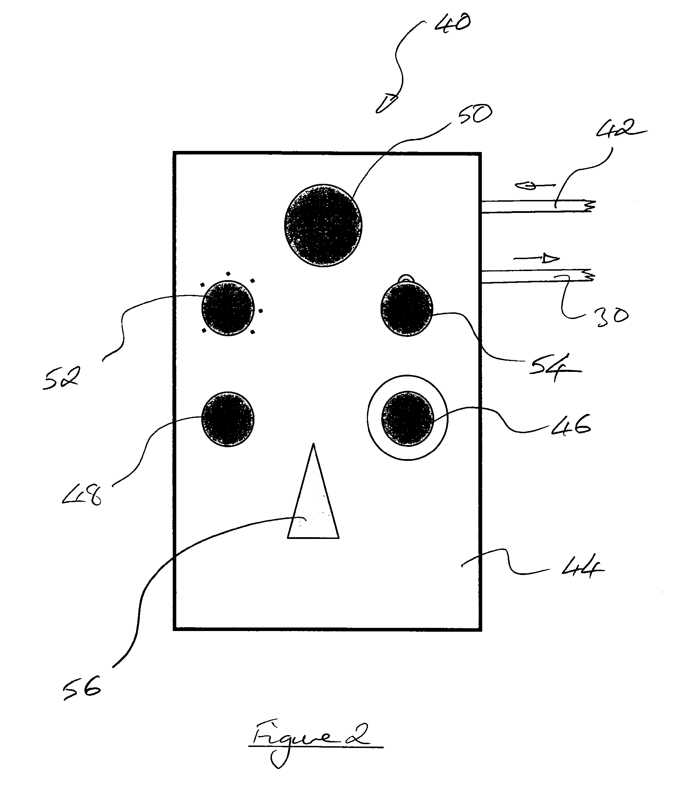

Figure 2 shows a controller 40 according to the present invention. Electrical power from the controller is conveyed to the LEDs 26, 28 of the glasses shown in Figures 1 a and 1 b by means of the signal cable 30. Electrical power is provided by means of a supply cable 42, which may be a mains cable should the controller be provided with a mains transformer or an appropriate direct current carrying cable should a direct current power source be available. Together, the glasses of Figures 1 a and 1 b and the controller of Figure 2 constitute photic stimulation apparatus according to the invention.

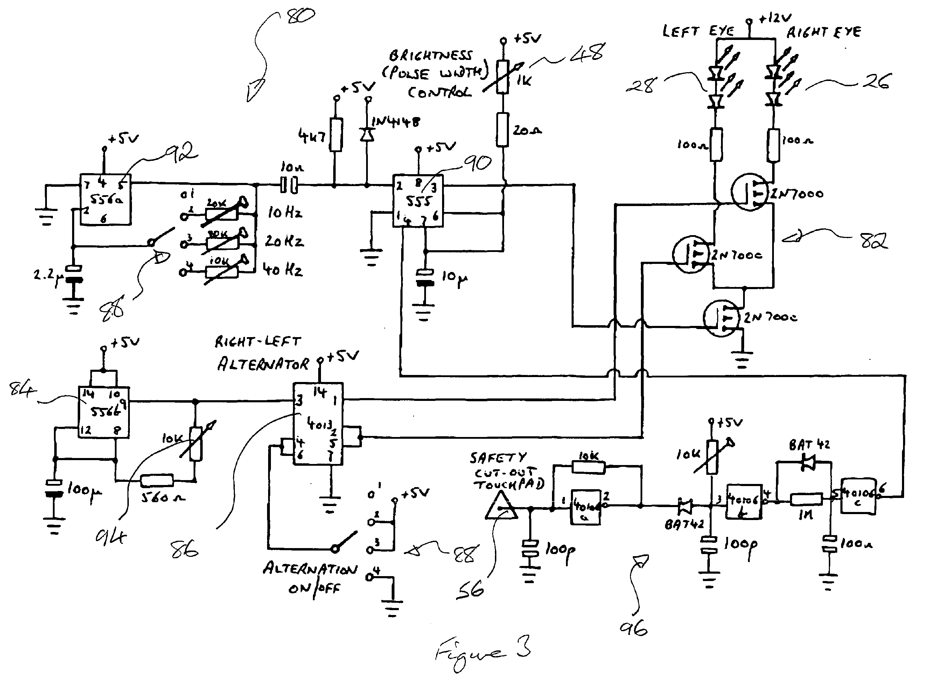

A plastics case 44 encloses the operative electronic components of the controller. The operative electronic components are described below with reference to Figure 3. Several controls are provided on the controller as follows. An on-off switch 46 provides the means for a user to turn on and turn off the apparatus. A rotary brightness control 48 provides for user control of the brightness of light emitted by the LEDs 26, 28 on the glasses 10. A rotary alternation frequency control 50 allows for user control of the frequency of alternation of pulsed light between the left and right eyes of the user of the glasses. The rotary brightness control 48 and the rotary alternation frequency control 50 are rotary potentiometers of conventional design. A pulse frequency control 52 allows a user to select one of six frequencies at which pulses of light are repeatedly emitted from each of the LEDs 26, 28. A mode control 54 allows a user to select one of four modes of operation. The pulse frequency control 52 and the mode control 54 are used in conjunction as described below in more detail. Each of the pulse frequency control 52 and the mode control 54 is a multi-way rotary switch of conventional design. The controller 40 also comprises a touch sensitive switch 56, which provides for safe operation of the apparatus as will be described below. The touch sensitive switch is a QT11 x touch sensor IC from Quantum Research Group Limited of Southampton, United Kingdom.

Figure 3 is a circuit schematic 80 of the LEDs 26, 28 mounted on the glasses 10 of Figures 1 a and 1 b and the electronic circuitry contained in the controller 40 of Figure 2. The right and left LEDs 26, 28 can be seen in the top right hand side of the schematic. The LEDs are biased and turned on and off by means of a resistor-transistor chain of conventional design 82. The gates of two of the transistors in the resistor-transistor chain 82 are driven by digital signals generated by one of two timers provided in a 556 timer integrated circuit (i.c.) 84, which is gated by a 4013 D-type bistable flip-flop 86. The 4013 D-type bistable flip-flop 86 is controlled by a three-pole, four way switch 88. The gate of one of the transistors in the resistor-transistor chain 82 is driven by a digital signal generated by a 555 timer 90, which is in turn driven by the other 92 of the two timers in the 556 timer i.c. The frequency of operation of the other 92 of the two timers in the 556 timer i.c. is set by means of the three-pole, four way switch 88. Thus, movement of the three-pole, four way switch 88 from one position to another controls the frequency of operation of the other 92 of the two timers in the 556 timer i.c. and gating of the 4013 D-type bistable flip-flop 86. Thus, at a first position the three-pole, four way switch 88 turns the power to the LEDs 26, 28 off. At a second position, there is no alternation between the first and second LEDs 26, 28 and the LEDs pulse repeatedly at 10 Hz. At a third position, there is no alternation between the first and second LEDs 26, 28 and the LEDs pulse repeatedly at 20 Hz. At a third position, there is alternation between the first and second LEDs 26, 28 and the LEDs pulse repeatedly at 40 Hz.

The reader will appreciate that structure of the control circuitry shown in Figure 3 is different to the arrangement of controls shown in Figure 2. The difference in structure will be clear from a reading of the description of operation provided below. In essence, the control circuitry of Figure 3 combines in the three-pole, four way switch 88 the functions of the on-off switch 46, the pulse frequency control 52 and the mode control 54 of Figure 2. The frequency of alternation between left and right LEDs 26, 28 can be altered by means of an alternation frequency potentiometer 94. Unlike the controller shown in Figure 2, which comprises a rotary alternation frequency control 50, there is no provision in the circuit according to Figure 3 for user change of frequency of alternation by means of alternation frequency potentiometer 94.

Returning to Figure 3, the amplitude of the control signal generated by the 555 timer i.c. 90 is controlled by means of the rotary brightness control 48 in the form of a potentiometer. Operation of the 555 timer 90 is gated by means of the touch sensitive switch 56. Signal conditioning circuitry 96 of conventional design conditions the output signal from the touch sensitive switch so that the signal is of appropriate form and amplitude to gate the 555 timer i.c. 90 properly.

The operation of the apparatus shown in Figures 1 a to 3 will now be described.

To begin an EMDR session a user brings the apparatus into use by donning the glasses. As described above, glasses formed of clear plastics are used where therapy is supervised. Different modes of operation of the apparatus are selected by means of the mode control 54 in the embodiment shown in Figure 2 or the three-pole, four way switch 88 in the embodiment shown in Figure 3. In a first mode, there is no alternation between left and right LEDs and both LEDs are pulsed at a frequency of between 10 Hz and 20 HZ under the control of the pulse frequency control 52. This mode is used to induce a sense of safety and a cognitive state of disengagement. In a second mode, there is no alternation between left and right LEDs and both LEDs are pulsed at a frequency of between 20 Hz and 40 HZ. This mode is used to engage and cognitively activate the user. In the first and second modes it is preferred that the user keeps his or her eyes closed. In a third mode, there is alternation between left and right LEDs at a frequency of 1 Hz and both LEDs are pulsed at a frequency of 40 HZ. This mode is used in an integration process in which temporal binding occurs as is described in Atkinson et al "Consciousness: mapping the theoretical landscape", Trends in Cognitive Sciences, 4, pp 372 to 382. In a fourth mode, there is alternation between left and right LEDs at a frequency according to the user's preference and no effective repeated pulsing of the LEDs. In practice the LEDs are pulsed at 80 Hz, which is a comparatively high frequency that is perceived by the user as being non-repeating. The brightness of the LEDs is set in accordance with the user's preference. This mode is used in a desensitisation phase. In the third and fourth modes of operation, the user may keep his or her eyes open or closed depending on his or her or the therapist's preference.

During use of the apparatus either the user or the therapist must maintain contact (e.g. with a finger) with the touch sensitive switch 56 to ensure that the apparatus is operative. Should contact with the touch sensitive switch be lost the apparatus will not operate. The touch sensitive switch 56 provides for safe operation. For example, the need to have contact with the switch provides of ease of discontinuation of therapy, e.g. if the user feels discomfort, and reduces the likelihood of improper use of the apparatus, e.g. by children.

The apparatus described herein can be used for a wide range of applications including relaxation, self-hypnosis, hypnosis and psychotherapy. The two means of stimulation, namely the repeated pulsing to each eye and the alternation between eyes, are believed to work together to regulate cognitive processing and to promote
inter-hemispherical integration to thereby effect an improvement in emotional processing. A repeated pulse rate of substantially 40 Hz has been found to be particularly effective at promoting psychological integration within the brain and the nervous system. Llinas, R and Ribary, U (1993) "Coherent 40 Hz oscillation characterises dream state in humans", Proc. National Academy of Science, 90, 2078 to 2081, describes the significance of 40 Hz as a key frequency in the functioning of the human brain. Thus, the present invention finds particular, but not exclusive application in neurodevelopmental disorders, such as Asperger's Syndrome and autism, in which there is improper emotional processing.

## Claims

1. Photic stimulation apparatus for stimulating the eyes of a subject, the apparatus comprising:
at least one light source that is operable to emit light, which, in use of the apparatus, is seen by each of the right and left eyes of the subject alternately and such that the light is seen at least mainly by one of the eyes each alternation; and
a controller operable: to provide light from the at least one light source to the right and left eyes alternately; and to pulse the at least one light source repeatedly at a frequency of between substantially 36 Hz and substantially 44 Hz when the at least one light source is seen by each eye during each alternation,
the photic stimulation apparatus being configured such that in use the photic stimulation apparatus is spaced apart from the eyeballs of the subject.

2. Apparatus according to claim 1, in which repeated pulsing of the at least one light source is at a frequency of substantially 40 Hz.

3. Apparatus according to any preceding claim, in which the controller is operable to provide light from the at least one light source to the right and left eyes alternately at a frequency of between substantially 0.25 Hz and substantially 5 Hz.

4. Apparatus according to any preceding claim, in which the controller is user operable to change at least one of an alternation frequency and a repeated pulse frequency.

5. Apparatus according to any preceding claim, in which the apparatus comprises: at least one first light source that is operable to emit light, which, in use of the apparatus, is seen at least mainly by a right eye of the right and left eyes of the subject; and at least one second light source that is operable to emit light, which, in use of the apparatus, is seen at least mainly by a left eye of the right and left eyes of the subject, and the controller is operable: to actuate the at least one first light source and the at least one second light source alternately; and to pulse an at least one light source repeatedly when the at least one light source is actuated.

6. Apparatus according to claim 5, in which the photic stimulation apparatus is configured such that, in use, the at least one first light source is disposed in front of the right eye of the user and the at least one second light source is disposed in front of the left eye of the user.

7. Apparatus according to claim 6, in which the photic stimulation apparatus is configured such that, in use, each of the at least one first and second light sources is disposed at a lateral periphery of the user's visual field.

8. Apparatus according to claim 7, in which the photic stimulation apparatus is configured such that, in use, light sources are disposed in the user's visual field solely at the lateral periphery of the user's visual field.

9. Apparatus according to any preceding claim, in which the photic stimulation apparatus comprises a support arrangement on which the at least one light source is mounted.

10. Apparatus according to claim 9, in which the support arrangement is configured to be mounted on the head of a user and the support arrangement is at least one of transparent and translucent such that, in use, a view of a face of user is substantially unobstructed by the support arrangement.

11. Apparatus according to claim 9 or 10, in which the support arrangement comprises glasses.

12. Apparatus according to claim 11, in which the photic stimulation apparatus comprises: at least a first light source mounted on a first leg of the glasses, the first leg being configured to engage with a right ear of the user; and at least a second light source mounted on a second leg of the glasses, the second leg being configured to engage with a left ear of the user.

13. Apparatus according to claim 12, in which each of the at least one first and second light sources is mounted on its respective leg such that, in use, light is emitted from the first and second light sources away from the eyes of a user.

14. Apparatus according to claim 13, in which a reflective component is mounted over each of a right lens and a left lens of the glasses such that a part of a field of view through each of the right and left lenses is obscured.

15. Apparatus according to claim 14, in which the reflective component is mounted such that it extends over a portion of a lens at a lateral periphery of the user's visual field.
